# EUROPEAN PATENT APPLICATION

(11) **EP 4 213 155 A1**
(43) Date of publication of application: **19.07.2023**
(21) Application number: 21866623.8
(22) Date of filing: 01.09.2021
(51) Int. Cl.: G16H 20/00

(54) **INFORMATION PROCESSING DEVICE, SUBJECT TERMINAL, INFORMATION PROCESSING SYSTEM, AND METHOD FOR CONTROLLING INFORMATION PROCESSING SYSTEM**

(30) Priority: 08.09.2020 JP 2020150767
(71) Applicant: Kyocera Corporation, Kyoto-shi Kyoto 612-8501 (JP)
(72) Inventor: IKEDA, Yutaka, Kyoto-shi, Kyoto 612-8501 (JP); NISHIZONO, Kazuhiro, Kyoto-shi, Kyoto 612-8501 (JP); ONISHI, Koji, Kyoto-shi, Kyoto 612-8501 (JP)
(74) Representative: TBK
(86) International application number: PCT/JP2021/032100
(87) International publication number: WO 2022/054664

(57) **Abstract**

Provided is an information processing device including: a measurement result acquiring unit configured to acquire a measurement result obtained by measuring a sample of a subject by using a measuring device; a history acquiring unit configured to acquire a history related to the subject; and a presentation information generation unit configured to generate information to be presented to the subject by using the measurement result of the subject, the history of the subject, a measurement result obtained by measuring a sample of another person, and a history related to the other person, and, according to the above information processing device, desirable information for a user may be generated.

## Description

### TECHNICAL FIELD

The present disclosure relates to an information processing device and the like configured to generate information to be presented to a subject by using a measurement result of the subject obtained through a measuring device, history information of the subject, and the like.

### BACKGROUND OF INVENTION

Patent Document 1 describes an information processing method in which physiological information of a user and correction information such as an amount of activity of the user are acquired and information to be provided to the user as a service is generated and output.

### CITATION LIST

### PATENT LITERATURE

Patent Document 1: JP 2018-113071 A

### SUMMARY

According to an aspect of the present disclosure, an information processing device includes: a measurement result acquiring unit configured to acquire a first measurement result obtained by measuring a sample of a subject by using a measuring device; a history acquiring unit configured to acquire a first history related to the subject; and an information generation unit configured to generate presentation information to be presented to the subject by using the first measurement result, the first history, a second measurement result obtained by measuring a sample of another person different from the subject, and a second history related to the other person.

According to an aspect of the present disclosure, an information processing device includes: a measurement result acquiring unit configured to acquire a first measurement result obtained by measuring a sample of a subject by using a measuring device; a history acquiring unit configured to acquire a first history related to the subject, the first history including a food purchase history related to food having been purchased by the subject; and an information generation unit configured to generate information to be presented to the subject by using the first person measurement result and the first history.

According to an aspect of the present disclosure, a subject terminal includes the information processing device and a presentation unit configured to present the information.

According to an aspect of the present disclosure, an information processing system includes the information processing device and the measuring device, and the measuring device and the information processing device are communicably connected through a wired or wireless dedicated line.

According to an aspect of the present disclosure, a control method of an information processing system includes: a step of acquiring a first measurement result obtained by measuring a sample of a subject by using a measuring device; a step of acquiring a first history related to the subject; a step of generating presentation information to be presented to the subject by using the first measurement result, the first history, a second measurement result obtained by measuring a sample of another person different from the subject, and a second history related to the other person; and a step of causing a presentation unit to present the presentation information.

According to an aspect of the present disclosure, a control method of an information processing system includes: acquiring a first measurement result obtained by measuring a sample of a subject by using a measuring device; acquiring a first history related to the subject, the first history including a food purchase history related to food having been purchased by the subject; generating information to be presented to the subject by using the first measurement result, the first history, and the food purchase history; and causing a presentation unit to present the information.

### BRIEF DESCRIPTION OF THE DRAWINGS

FIG. 1 is a conceptual diagram illustrating an overall overview of an information processing system according to an embodiment of the present disclosure.
FIG. 2 is a functional block diagram illustrating a main portion configuration of each of constituent elements included in an information processing system.
FIG. 3 is a diagram illustrating an example of subject history information stored in a storage of an information processing device.
FIG. 4 is a diagram illustrating an example of subject measurement result information stored in a storage of an information processing device.
FIG. 5 is a diagram illustrating an example of presentation information data stored in a storage of an information processing device.
FIG. 6 is a diagram illustrating an example of presenting presentation information in a subject terminal.
FIG. 7 is a flowchart illustrating an example of a process flow in an information processing system.
FIG. 8 is a functional block diagram illustrating a main portion configuration of an information processing device according to another embodiment of the present disclosure.
FIG. 9 is a diagram illustrating an example of subject history information stored in a storage of an information processing device in the other embodiment.
FIG. 10 is a flowchart illustrating an example of a process flow of an information processing system in the other embodiment.
FIG. 11 is a functional block diagram illustrating a main portion configuration of a subject terminal in still another embodiment.

### DESCRIPTION OF EMBODIMENTS

### First Embodiment

### Overview of Information Processing System 1

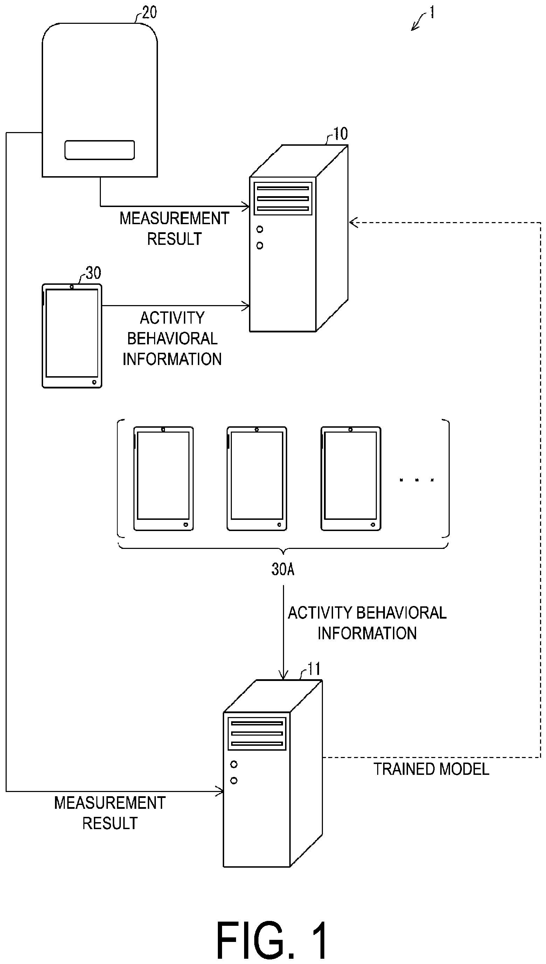

An embodiment of the present disclosure will be described in detail below. First, an overview of an information processing system 1 according to the present embodiment will be described with reference to FIG. 1. FIG. 1 is a conceptual diagram illustrating the overview of the information processing system 1.

As illustrated in FIG. 1, the information processing system 1 according to the present embodiment includes an information processing device 10, a measuring device 20, and a subject terminal 30. A trained model generation device 11 and an other-person terminal 30A are also depicted in FIG. 1, but these are allowed not to be included in the information processing system 1. The information processing device 10 and the measuring device 20 may communicate with each other through a communication line, and the information processing device 10 and the subject terminal 30 may also communicate with each other through a communication line. The measuring device 20 and the subject terminal 30 may communicate with each other in a wireless or wired communication form. The information processing device 10 and the measuring device 20 may communicate with each other through a dedicated line. The other-person terminal 30A carried by another person different from the subject may also communicate with the information processing device 10. When the measuring device 20 is a device configured to acquire physiological information directly from a subject, the measuring device 20 may be incorporated in the subject terminal 30. When the information processing system 1 includes the trained model generation device 11, the trained model generation device 11 may also communicate with the information processing device 10.

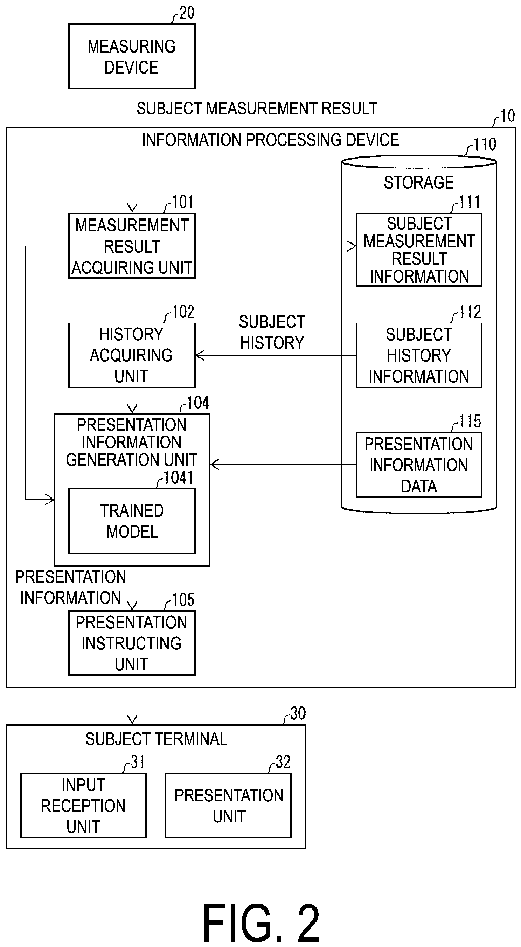

In the information processing system 1, the physiological information of the subject is measured by the measuring device 20, and a subject measurement result obtained by the measurement is transmitted to the information processing device 10. The subject measurement result is also referred to as a first measurement result. The measuring device 20 may use a sample derived from a subject as a measurement target or may acquire physiological information of a subject directly from the subject. The subject terminal 30 measures activity behavioral information which is the information related to the activity and behavior of the subject or receives input of the activity behavioral information from the subject. The subject terminal 30 transmits the measured or received activity behavioral information to the information processing device 10. When the subject terminal 30 measures the activity behavioral information, the subject terminal 30 functions as a measuring device configured to measure the activity behavioral information of the subject. In the information processing device 10, the measurement result is stored as subject measurement result information 111, and the activity behavioral information is stored as subject history information 112 (see FIG. 2).

The measuring device 20 measures physiological information of the other person. The measuring device 20 for acquiring the physiological information of the subject may differ from the measuring device 20 for acquiring the physiological information of the other person. The other-person terminal 30A measures the activity behavioral information which is the information related to the activity and behavior of the subject or receives input of the activity behavioral information from the other person. When the measuring device 20 is a device configured to acquire the physiological information directly from the other person, the measuring device 20 may be incorporated in the other-person terminal 30A. The types of the physiological information and the activity behavioral information relating to the subject correspond to the types of the physiological information and the activity behavioral information relating to the other person.

When a measurement result of the subject is transmitted from the measuring device 20, the information processing device 10 generates presentation information to be presented to the subject by using a trained model. The trained model may be a model trained by using a set of (i) each measurement result and history information (for example, activity behavioral information) of the other person and (ii) execution information, as teaching data. The execution information may include, for example, information on food or medicine to be preferably ingested or ingestion forms thereof, information on exercises to be preferably performed, and information on preferable sleep.

To obtain the teaching data as described above, an implementor of a trained model 1041 acquires big data indicating (i) measurement results of many other persons, (ii) histories of the other persons (other-person histories), (iii) execution information indicating matters performed by the other persons after the acquisition of the measurement results and the other-person histories, and (iv) effects of performing the above matters. The implementor may extract a large number of sets of (i), (ii), and (iii) from which favorable effects (iv) have been obtained and may utilize aggregation of these sets as the teaching data. The other-person histories are each also referred to as a second history.

The trained model generation device 11 may construct the trained model 1041 by causing an untrained learning model to perform machine learning using the above-described teaching data. The specific method for the machine learning is not particularly limited.

In a case where the trained model 1041 is generated in the trained model generation device 11, the trained model generation device 11 may acquire big data including all of the above (i) to (iv) serving as a source of the teaching data from another device. The trained model generation device 11 may acquire each of (i) to (iv) from different devices and associate the data of (i) to (iv) with one another to obtain big data. By using date information included in the respective data, for example, (i) to (iv) may be associated with one another.

In the case where each of (i) to (iv) is acquired from different devices, (i) the measurement results of many other persons may be obtained from the measuring device 20 when the trained model generation device 11 and the measuring device 20 are communicably connected to each other, for example. When the trained model generation device 11 and the other-person terminals 30A are communicably connected, (ii) history information of the other persons may be obtained from the other-person terminals 30A.

When a device storing the data of (iii) and (iv) and the trained model generation device 11 are communicably connected, the data of (iii) and (iv) may also be acquired from the above device.

The information processing device 10 inputs the subject measurement result information 111 and the subject history information 112 to the trained model 1041 and obtains execution information as output thereof. The execution information is related to matters to be preferably performed by the subject. The execution information refers to, for example, information on food or medicine to be preferably ingested by the subject or ingestion forms thereof, information on exercises to be preferably performed by the subject, and information on sleep preferable for the subject. In this manner, the trained model 1041 specifies, as a matter preferable for the subject, a matter that was performed by the other person having the same measurement result and history information as the measurement result and history information of the subject and exhibited a preferable effect. Examples of the information on food include information on common food and information on health food. Examples of the common food include vegetable food including vegetables and fruit, and animal food including dairy products and meat. The health food is expected to have an effect of improving health, and examples thereof include food in which specific nutrients are added to common food, and supplements.

The information processing device 10 generates presentation information to be presented to the subject by using the execution information and presents the presentation information on the subject terminal 30. The presentation information may be the execution information itself or may include other information to be preferably presented to the subject in addition to the execution information.

As described above, the information to be presented to the subject is generated based on the matters that were performed by the other person having the same measurement result, same activity behavioral information, and the like as those of the subject and exhibited preferable effects. Thus, the information processing device 10 is able to generate information appropriate for the subject.

The trained model generation device 11 generates the trained model described above. The trained model generation device 11 may be incorporated in the information processing device 10 or may be a device separated from the information processing device 10. The trained model generation device 11 generates the trained model by using the teaching data described above.

When the trained model generation device 11 and the information processing device 10 are separate devices, the trained model generated by the trained model generation device 11 is incorporated in the information processing device 10 in advance.

### Details of Information Processing System 1

Each of the constituent elements included in the information processing system 1 will be described in detail with reference to FIG. 2. FIG. 2 is a functional block diagram illustrating a main portion configuration of each of the constituent elements included in the information processing system 1.

The measuring device 20 measures a sample of a subject and transmits a measurement result to the information processing device 10. Communication between the measuring device 20 and the information processing device 10 may be carried out using a known network and may be carried out by wired or wireless communication. The communication line may be a dedicated line, or may be, for example, a network using an LPWA (low power wide area network).

Examples of contents measured by the measuring device 20 include the concentration of a specific substance contained in blood or urine as a sample. Since the concentration of the specific substance in the sample can be measured using a known technique, the description of the concentration measurement will be omitted.

The information processing device 10 includes a measurement result acquiring unit 101, a history acquiring unit 102, a presentation information generation unit (information generation unit) 104, a presentation instructing unit 105, and a storage 110. The storage 110 may be provided separate from the information processing device 10.

The measurement result acquiring unit 101 acquires a measurement result obtained by the measuring device 20 and outputs the acquired measurement result to the presentation information generation unit 104. Further, the measurement result acquiring unit 101 stores the acquired measurement result in the storage 110 as the subject measurement result information 111.

The subject measurement result information 111 is an accumulation of the measurement results transmitted from the measuring device 20 to the information processing device 10 each time the subject measures a sample with the measuring device 20.

When the measurement result acquiring unit 101 acquires the measurement result of the subject, the history acquiring unit 102 acquires the subject history from the subject history information 112 stored in the storage 110 and outputs the acquired subject history to the presentation information generation unit 104. The subject history is also referred to as a first history.

As described above, the subject history information 112 is the activity behavioral information of the subject and the activity behavioral information of the other person.

Examples of the activity behavioral information include any of meal contents, meal times, exercise contents, exercise time, sleeping time, heart rate, body temperature, wake-up time, and bedtime of the subject or the other person, and a combination thereof. These pieces of information are measured or input by the subject terminal 30, thereby being transmitted from the subject terminal 30 to the information processing device 10. These pieces of information may be transmitted by a function of a specific application installed in the subject terminal 30.

As described above, the subject history information 112 and the other-person history information may include information on a lifestyle habit or a biological cycle indicated by any of meal contents, meal times, exercise contents, exercise time, sleeping time, heart rate, body temperature, wake-up time, and bedtime of the subject or the other person, or a combination thereof. Thus, the presentation information generation unit 104 is able to generate information in accordance with the lifestyle habit or the physiological state of the subject.

The presentation information generation unit 104 inputs the measurement result acquired by the measurement result acquiring unit 101 and the subject history acquired by the history acquiring unit 102 to the trained model 1041 and generates presentation information by using the execution information obtained from the trained model 1041. The trained model 1041 is generated by machine learning. The algorithm for machine learning is not particularly limited. For example, a machine learning algorithm such as a neural network may be used, and particularly, machine learning by a neural network having experienced deep learning may be performed.

As described above, the teaching data for machine learning may be a set of the "other-person measurement result and other-person history" and the "execution information". Since the trained model 1041 is trained using the above teaching data, when the "subject measurement result and subject history" are input, the trained model 1041 can output "execution information" suitable for the input. The presentation information generation unit 104 may generate the presentation information using the execution information. The presentation information generation unit 104 stores the trained model 1041 having experienced machine learning using the above-discussed teaching data. The other-person measurement result is also referred to as a second measurement result.

As described above, the presentation information generation unit 104 inputs the subject measurement result and the subject history to the trained model 1041 and generates the presentation information by obtaining the execution information from the trained model 1041.

In other words, the presentation information generation unit 104 uses the trained model 1041 to generate the presentation information corresponding to the subject measurement result and the subject history. The trained model 1041 can be considered as a model having experienced a learning process based on the relationship between the other-person measurement result and other-person history, and the execution information related to the matters performed by the other person after the acquisition of the other-person measurement result and other-person history.

Examples of the presentation information generated by the presentation information generation unit 104 include food information, medicine information, information on exercises, and information on sleep.

For example, when food information or medicine information is to be generated as presentation information, the presentation information generation unit 104 obtains execution information including ingestion forms such as types thereof (including health food), an ingestion amount at a time, and an ingestion frequency from the trained model 1041, and generates the presentation information. When information on exercises is to be generated as presentation information, the presentation information generation unit 104 obtains execution information including exercise contents, time, and frequency from the trained model 1041, and generates the presentation information.

The presentation information generation unit 104 may generate presentation information including, in addition to these pieces of information, information indicating a subsequent measurement time using the measuring device 20. The subsequent measurement time may be indicated as a specific time or may be indicated as a measurement frequency. The subsequent measurement time may be calculated by adding the number of days predetermined in accordance with the type of measurement to the latest measurement date or may be calculated based on a predetermined table indicating a correspondence relationship between the evaluation of the measurement result and the subsequent measurement date. The measurement frequency may be determined in advance in accordance with the type of measurement or may be calculated based on a predetermined table indicating a correspondence relationship between the evaluation of the measurement result and the measurement frequency.

The presentation information generation unit 104 may generate presentation information including information indicating a measurement method different from the method by which the sample is measured using the measuring device 20. For example, when the measurement result obtained by the measuring device 20 does not fall within a predetermined numerical value range, the presentation information generation unit 104 may determine that the measurement has not been normally carried out and may include a message prompting the subject to carry out the measurement by another measurement method or another measuring device in the presentation information.

The presentation information generation unit 104 may generate, as the presentation information, information that is not related to the measurement result of the subject but is specified based on the subject history. In this case, a trained model having been trained using, as teaching data, a relationship between the "other-person history" and the execution information (for example, information on ingested health food) effective when performed by the other person, is allowed to be used as a model different from the trained model 1041. The presentation information generation unit 104 may select a relevant health food from a health food list registered in advance, based on the subject history, and generate information related to the selected health food as the presentation information without using the trained model 1041.

The presentation information generation unit 104 may include information indicating a result obtained by comparing the measurement results of the subject and the other person in the presentation information. For example, the presentation information generation unit 104 may generate presentation information with which the transition of the measurement result of the other person and the transition of the measurement result of the subject can be confirmed on the list.

When the presentation information generation unit 104 generates food information or the like, information recommending regular purchase of the food may be included in the presentation information. For example, the presentation information generation unit 104 may generate presentation information indicating, to the subject, that a discount is given by regularly purchasing a certain food as compared to a case where the food is purchased as needed.

The trained model 1041 may be updated based on the presentation information generated by the presentation information generation unit 104.

As described above, the presentation information generated by the presentation information generation unit 104 may include the ingestion form of food including health food. Thus, the ingestion form of health food may be presented to the subject.

The presentation information generated by the presentation information generation unit 104 may include at least any of information on food, information on exercises, and information on sleep. Thus, the information on food, the information on exercises, and the information on sleep may be presented to the subject.

The presentation information generation unit 104 may include, in the presentation information, information indicating a preferred time for the subject to perform the subsequent measurement using the measuring device 20. Thus, the subsequent measurement time may be presented to the subject.

The presentation information generation unit 104 may include, in the presentation information, information indicating a method of measurement to be carried out by the subject different from the method of measurement using the measuring device 20. Thus, the subject may be presented to carry out the measurement by the different measurement method.

The presentation information generated by the presentation information generation unit 104 may also include information on the food that is specified based on the subject history and is not related to the subject measurement result. Thus, the information on the food that is not related to the measurement result based on the subject history may be generated for the subject.

The presentation information generation unit 104 may include information indicating a result obtained by comparing the measurement results of the subject and the other person in the presentation information. Thus, the comparison of the measurement results with the other person may be presented to the subject.

When the presentation information generation unit 104 presents information on food, information recommending regular purchase of the food may be included in the presentation information. Thus, the subject may be prompted to regularly purchase health food.

The presentation instructing unit 105 acquires the presentation information generated by the presentation information generation unit 104, transmits the acquired presentation information to the subject terminal 30, and causes the subject terminal 30 to present the presentation information.

The information processing device 10 includes the presentation instructing unit 105 capable of communicating with the subject terminal 30 capable of inputting or measuring the information to be included in the subject history information 112 and configured to cause a presentation unit 32 of the subject terminal 30 to present the presentation information. Thus, the information desirable for the subject may be presented.

The storage 110 stores the subject measurement result information 111, the subject history information 112, and presentation information data 115. By providing the storage 110 in the information processing device 10, the subject measurement result can be stored within the information processing system 1. The storage 110 may store the measurement results and histories related to the other persons.

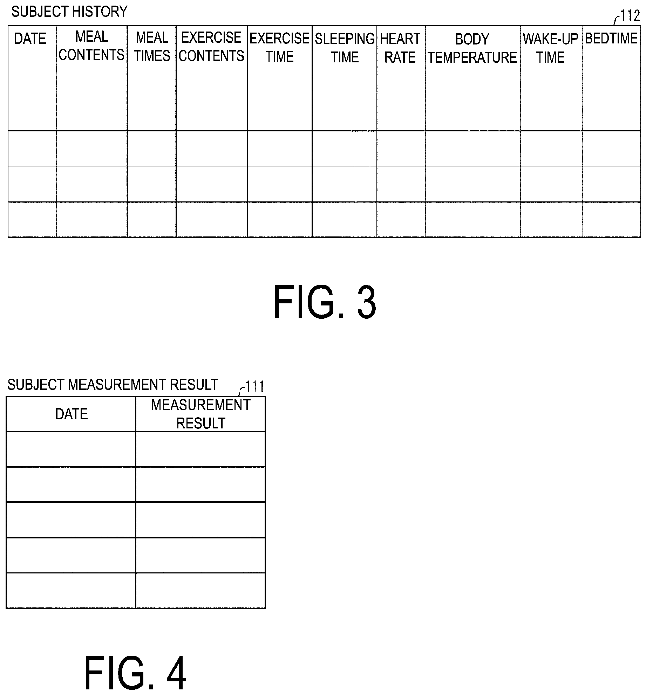

FIG. 3 to FIG. 5 illustrate examples of the subject measurement result information 111, the subject history information 112, and the presentation information data 115 (presentation sentence data 115A and a presentation information group 115B) stored in the storage 110.

As illustrated in FIG. 3, in the subject history information 112, for example, meal contents, meal times, exercise contents, exercise time, sleeping time, heart rate, body temperature, wake-up time, and bedtime regarding the subject are recorded being associated with dates. Not all of these pieces of information are essential; only some of the pieces of information may be recorded, and information other than these pieces of information may be recorded. The history information of the subject corresponds to the history information of the other person, and therefore, for example, meal contents, meal times, exercise contents, exercise time, sleeping time, heart rate, body temperature, wake-up time, and bedtime regarding the other person are also recorded in the other-person history information being associated with dates.

As illustrated in FIG. 4, in the subject measurement result information 111, the measurement result of the subject by the measuring device 20 and the date of the measurement are recorded being associated with each other. Similar to the subject measurement result information 111, in the other-person measurement result information, the measurement result of the other person by the measuring device 20 and the date of the measurement are also recorded being associated with each other.

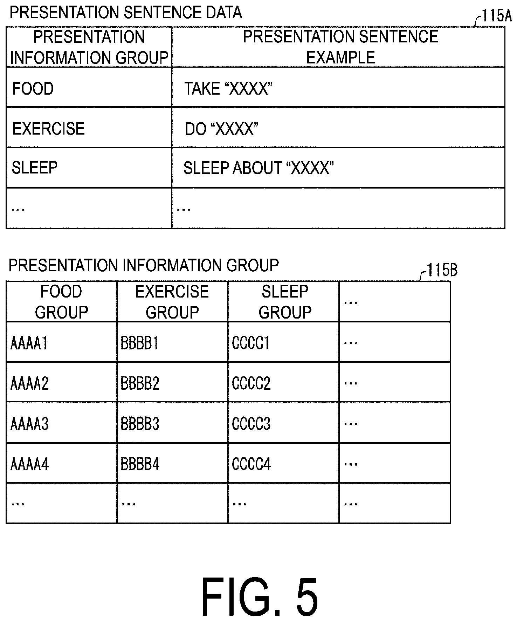

The presentation information data 115 corresponds to a template used when the presentation instructing unit 105 generates the presentation information. As illustrated in FIG. 5, the presentation information data 115 includes the presentation sentence data 115A, in which presentation information groups and presentation sentence examples are associated with each other, and the presentation information group 115B including contents of each presentation information group. As illustrated in the presentation information group 115B, the presentation information groups include, for example, a food group, an exercise group and a sleep group, and the contents of each group are recorded. The presentation information groups may include a medicine group. As the food group, for example, a common food group may be recorded, a health food group may be recorded, or the common food group and health food group may be recorded. The presentation information groups are required to record the contents of at least one group. In the example illustrated in FIG. 5, "AAAA1", "AAAA2", and the like are recorded as the food group, and "BBBB1", "BBBB2", and the like are recorded as the exercise group.

In the presentation sentence data 115A, the presentation information groups and the presentation sentence examples are recorded. For example, when "AAAA1" of the food group is to be presented, the presentation instructing unit 105 may generate presentation information saying "Take "AAAA1"".

The food group may include health food, and the presentation sentence example may indicate an ingestion frequency instead of simply saying "Take". For example, the presentation information may be "Take health food XXXX once a day".

The subject terminal 30 is a mobile terminal carried by the subject and includes an input reception unit 31 and the presentation unit 32. The input reception unit 31 is an interface for receiving input to the subject terminal 30, and is, for example, entry buttons of characters or the like. The presentation unit 32 presents various types of information, and is, for example, a display. A touch panel may be provided, and the touch panel may serve as both the input reception unit 31 and the presentation unit 32.

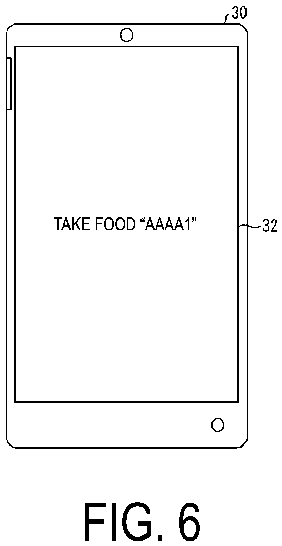

FIG. 6 illustrates a presentation example of presentation information in the subject terminal 30. In the example illustrated in FIG. 6, "Take food "AAAA1"" is presented as presentation information on the presentation unit 32.

The subject terminal 30 may measure or input information related to activity behavioral information of the subject. For example, in the case of the activity behavioral information mentioned above, the subject inputs meal contents, meal times, exercise contents, exercise time, sleeping time, wake-up time, bedtime, and the like. Heart rate and body temperature may be measured by a measuring device capable of measuring them, and the measured values may be input by the subject. Alternatively, a sensor may be provided in the subject terminal 30, and the heart rate and body temperature may be directly measured.

### Process Flow in Information Processing System 1

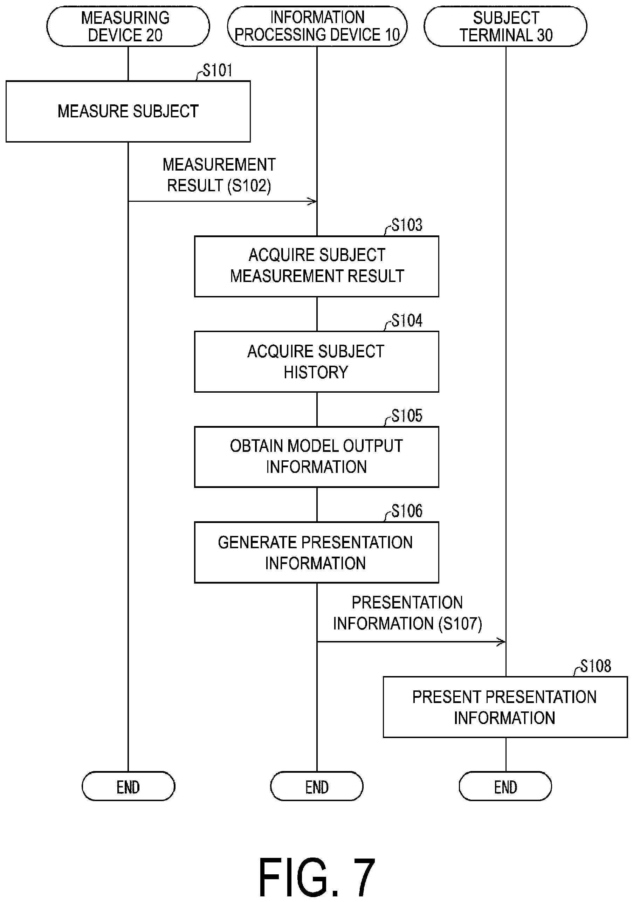

A process flow in the information processing system 1 will be described with reference to FIG. 7. FIG. 7 is a flowchart illustrating the process flow in the information processing system 1.

As illustrated in FIG. 7, in the information processing system 1, a sample of a subject is measured first by the measuring device 20 (S101). The measurement result of the subject measured by the measuring device 20 is transmitted to the information processing device 10 (S102). When the information processing device 10 acquires the measurement result of the subject from the measuring device 20 (S103), the history acquiring unit 102 acquires the subject history information 112 of the subject from the storage 110 (S104).

The presentation information generation unit 104 inputs the measurement result of the subject and the subject history information 112 to the trained model 1041 to obtain model output information (S105). The presentation information generation unit 104 generates presentation information from the obtained model output information (S106).

The presentation instructing unit 105 transmits the presentation information generated by the presentation information generation unit 104 to the subject terminal 30 (S107), and the transmitted presentation information is presented on the presentation unit 32 of the subject terminal 30 (S108).

As described above, the information processing device 10 according to the present embodiment uses a trained model having been trained using teaching data including an other-person measurement result as the second measurement result obtained by measuring a sample of the other person different from the subject and also including the other-person history as the second history related to the other person, and generates presentation information from the subject measurement result as the first measurement result obtained by measuring a sample of the subject and the first history related to the subject.

In other words, the information processing device 10 according to the present embodiment includes the measurement result acquiring unit 101 configured to acquire the subject measurement result as the first measurement result obtained by measuring a sample of the subject by using the measuring device 20; the history acquiring unit 102 configured to acquire the subject history as the first history related to the subject; and the presentation information generation unit 104 configured to generate execution information corresponding to the first measurement result and the first history based on the relationship between (i) the second measurement result obtained by measuring the other person different from the subject and the second history related to the other person and (ii) the execution information on the matters performed by the other person, and generate information to be presented to the subject by using the generated execution information.

As a result, the presentation information generation unit 104 may generate the presentation information by using not only the measurement result and the history related to the subject but also the measurement result and the history related to the other person, and therefore may generate more desirable information for the subject.

In the above-described embodiment, an example in which the presentation information is generated using the trained model 1041 is described. The present disclosure is not limited thereto, and the presentation information may be generated by another algorithm.

For example, a table is provided in advance in the information processing device 10, in which the other-person measurement results and the other-person histories, and the execution information indicating the contents to be preferably performed by the other persons with respect to the combinations of the other-person measurement results and the other-person histories. When the measurement result acquiring unit 101 acquires the measurement result of the subject, the presentation information generation unit 104 combines the acquired subject measurement result with the stored subject history. Further, the presentation information generation unit 104 extracts a combination of the other-person measurement result and the other-person history closest to the above-described combination. The presentation information is generated using the execution information corresponding to the extracted combination. Thus, the information desirable for the subject may be generated without generating the trained model 1041.

### Second Embodiment

Another embodiment of the present disclosure will be described below. Note that, for convenience of description, a member having the same function as that of a member described in the embodiments described above is denoted by the same reference sign, and description thereof will not be repeated.

An information processing device 10A according to the present embodiment is different from the information processing device 10 of the first embodiment described above in that the information processing device 10A generates presentation information using a purchase history of a subject. In the present embodiment, information of another person may or may not be used.

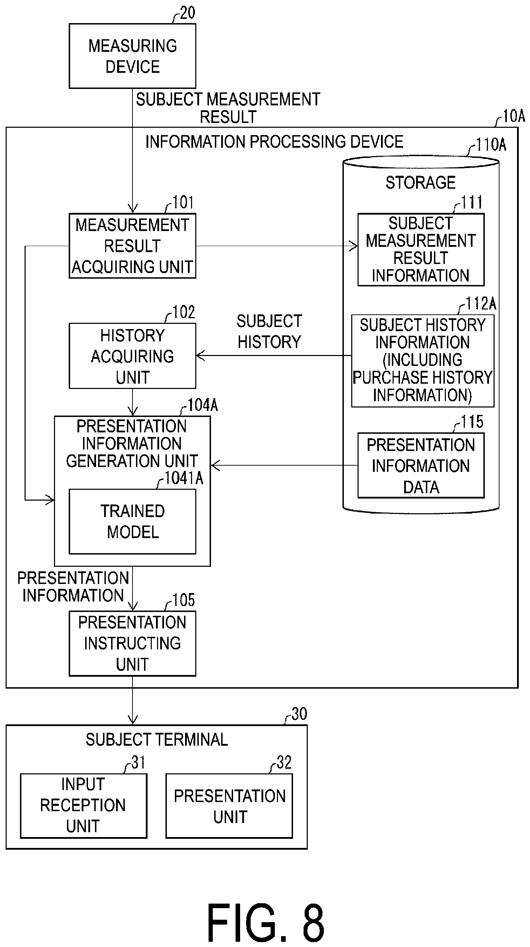

FIG. 8 illustrates a main portion configuration of the information processing device 10A according to the present embodiment. FIG. 8 is a functional block diagram illustrating the main portion configuration of the information processing device 10A.

As illustrated in FIG. 8, the information processing device 10A includes a measurement result acquiring unit 101, a history acquiring unit 102, a presentation information generation unit 104, a presentation instructing unit 105, and a storage 110A. As illustrated in FIG. 8, the storage 110A stores subject measurement result information 111, subject history information 112A, and presentation information data 115.

In the subject history information 112A, information of a purchase history related to food of the subject is stored in addition to the information of the subject history information 112.

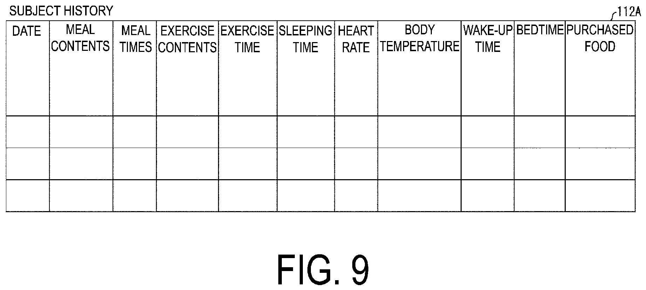

FIG. 9 illustrates an example of the subject history information 112A. As illustrated in FIG. 9, in the subject history information 112A, food purchased by the subject and dates of the purchase are recorded being associated with each other.

The presentation information generation unit 104 generates presentation information by using the subject history information 112A including a measurement history and the purchase history of the subject, and also using the presentation information data 115.

In other words, the presentation information generation unit 104 of the present embodiment uses a trained model 1041A generated by machine learning to generate the presentation information from the subject measurement result and the subject history (including the purchase history).

The trained model 1041A is generated by being trained using a set of the subject measurement result and history information and the execution information, as teaching data. As described above, the execution information may include, for example, information on food or medicine to be preferably ingested or ingestion forms thereof, information on exercises to be preferably performed, and information on preferable sleep. In the present embodiment, the execution information may be information indicating matters performed by the other person or may be information indicating matters performed by the subject. Hereinafter, an example of using information indicating matters performed by the other person as the execution information will be described.

To obtain such teaching data, an implementor of the trained model 1041A acquires big data indicating (i) measurement results of many other persons, (ii) histories of the other persons (other-person histories), (iii) execution information indicating matters performed by the other persons after the acquisition of the measurement results and the other-person histories, and (iv) effects of performing the above matters. The implementor may extract a large number of sets of (i), (ii), and (iii) from which favorable effects (iv) have been obtained and may utilize aggregation of these sets as the teaching data.

With this, the presentation information generation unit 104 may generate appropriate presentation information by taking the subject measurement result and the subject history as inputs.

Since the presentation information is generated by also using the purchase history related to the food of the subject, appropriate presentation information may be generated. This is because the presentation information may be generated using the relationship between the food purchased by the subject in the past and the measurement result after the purchase.

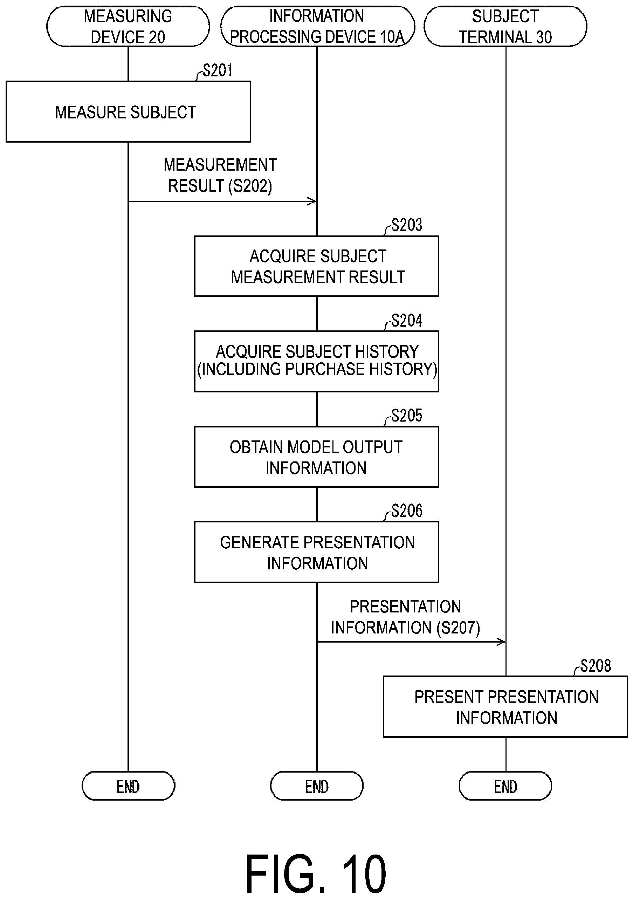

A process flow in the information processing system 1 according to the present embodiment will be described with reference to FIG. 10. FIG. 10 is a flowchart illustrating the process flow in the information processing system 1 of the present embodiment.

As illustrated in FIG. 10, in the information processing system 1 according to the present embodiment, a sample of the subject is measured first by the measuring device 20 (S201). The measurement result of the subject measured by the measuring device 20 is transmitted to the information processing device 10A (S202). When the information processing device 10 acquires the measurement result of the subject from the measuring device 20 (S203), the history acquiring unit 102 acquires the subject history information 112A including the purchase history of the subject from the storage 110 (S204).

The presentation information generation unit 104 inputs the measurement result of the subject and the subject history information 112A to the trained model 1041 to obtain model output information (S205). Subsequently, the presentation information generation unit 104 generates presentation information from the obtained model output information by using the presentation information data 115 (S206).

The generated presentation information is transmitted by the presentation instructing unit 105 to a subject terminal 30 (S207) and is presented on a presentation unit 32 of the subject terminal 30 (S208).

As discussed above, the information processing device 10A according to the present embodiment includes the measurement result acquiring unit 101 configured to acquire the subject measurement result as the first measurement result obtained by measuring a sample of the subject by using the measuring device 20; and the history acquiring unit 102 configured to acquire the subject history as a first history related to the subject. The first history includes a food purchase history related to food purchased by the subject. The information processing device 10A according to the present embodiment further includes the presentation information generation unit 104 configured to generate information to be presented to the subject by using the first measurement result and the first history.

As a result, the presentation information generation unit 104 may generate the presentation information by using not only the measurement result and history related to the subject but also the food purchase history of the subject, and therefore may generate more desirable information for the subject.

### Third Embodiment

Another embodiment of the present disclosure will be described below. Note that, for convenience of description, a member having the same function as that of a member described in the embodiments described above is denoted by the same reference sign, and description thereof will not be repeated.

The processing carried out by the information processing device 10 in the first embodiment is carried out by a subject terminal 30X in the present embodiment. That is, the subject terminal 30X alone may generate and present presentation information.

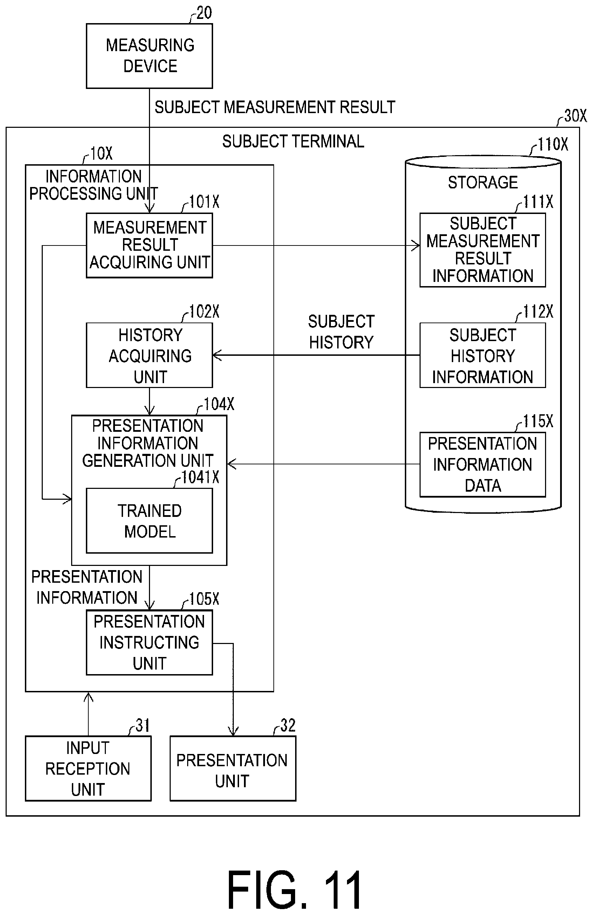

FIG. 11 illustrates a main portion configuration of the subject terminal 30X according to the present embodiment. FIG. 11 is a functional block diagram illustrating a main portion configuration of the subject terminal 30X.

As illustrated in FIG. 11, the subject terminal 30X includes an information processing unit 10X, a storage 110X, an input reception unit 31, and a presentation unit 32.

The information processing unit 10X includes a measurement result acquiring unit 101X, a history acquiring unit 102X, a presentation information generation unit 104X, and a presentation instructing unit 105X; the functions thereof are the same as those of the measurement result acquiring unit 101, the history acquiring unit 102, the presentation information generation unit 104, and the presentation instructing unit 105 of the first embodiment, respectively. That is, the subject terminals 30X include the functions of the information processing device 10 in the first embodiment.

The storage 110X stores subject measurement result information 111X, subject history information 112X, and presentation information data 115X. These pieces of information and data are the same as the subject measurement result information 111, the subject history information 112, and the presentation information data 115 in the first embodiment.

As described above, in the present embodiment, by including the functions of the information processing device 10 in the subject terminal 30X, the subject terminal 30X alone may generate and present the presentation information.

As in the information processing device 10 of the first embodiment, the storage 110X may store information on other persons (other-person history information and other-person measurement result information). In this case, these pieces of information may be acquired directly from the other-person terminals 30A or may be acquired from a server or the like on a network in which these pieces of information are stored.

As described above, the subject terminal 30X according to the present embodiment includes the information processing unit 10X and the presentation unit 32. The information processing unit 10X includes the measurement result acquiring unit 101, the history acquiring unit 102, and the presentation information generation unit 104. The measurement result acquiring unit 101 acquires a subject measurement result as a first measurement result obtained by measuring a sample of the subject by using a measuring device 20. The history acquiring unit 102 acquires a subject history as a first history related to the subject. The presentation information generation unit 104 generates presentation information to be presented to the subject by using the first measurement result, the first history, a second measurement result obtained by measuring a sample of another person different from the subject, and a second history related to the other person.

Thus, the subject terminal 30X alone may present information to the subject by using the subject measurement result, the subject history, the other-person measurement result, and the other-person history.

Further, the subject terminal 30X includes the storage 110X configured to store the subject measurement result as the first measurement result. Thus, the subject measurement result may be stored in the subject terminal 30X.

Similar to the information processing device 10A of the second embodiment, the subject terminal 30X may include, in the first history, a food purchase history related to food purchased by the subject. As a result, the presentation information generation unit 104X may generate the presentation information by using not only the measurement result and history related to the subject but also the food purchase history of the subject, and therefore may generate more desirable information for the subject.

### Example of Software Implementation

Control blocks of the information processing device 10 and the information processing unit 10X (particularly, the measurement result acquiring units 101 and 101X, the history acquiring units 102 and 102X, other-person information acquiring units 103 and 103X, the presentation information generation units 104 and 104X, and the presentation instructing units 105 and 105X) may be implemented by logic circuits (hardware) formed in integrated circuits (IC chips) or the like, or may be implemented by software.

In the latter case, the information processing device 10 and the information processing unit 10X each include a computer configured to execute instructions of a program that is software implementing each of the functions. The computer includes, for example, at least one processor (control device) and at least one computer-readable recording medium storing therein the above-mentioned program. In the computer, the processor reads the above program from the recording medium and executes the read program to achieve an object of the present disclosure. As the processor, a central processing unit (CPU) can be used, for example. As the recording medium, a "non-transitory tangible medium" such as, for example, a read only memory (ROM), a tape, a disk, a card, a semiconductor memory, a programmable logic circuit, and the like can be used. Additionally, a random access memory (RAM) for loading the above program may be further provided. The above program may be supplied to the computer via any transmission medium (a communication network, broadcast wave, or the like) capable of transmitting the program. Further, one aspect of the present disclosure may be implemented in the form of data signals embedded in a carrier wave in which the above program is embodied by electronic transmission.

As described above, the information processing device 10 according to each aspect of the present disclosure may be achieved by a computer. In a case of achieving the information processing device 10 by a computer, a control program of the information processing device 10 for causing the computer to operate as constituent elements (software elements) included in the information processing device 10 to achieve the information processing device 10 by the computer, and a computer-readable recording medium storing therein the above control program also fall within the scope of the present disclosure. The information processing unit 10X as well is the same as the information processing device 10 according to each aspect of the present disclosure.

In the present disclosure, the invention has been described above based on the various drawings and examples. However, the invention according to the present disclosure is not limited to each embodiment described above. That is, the embodiments of the invention according to the present disclosure can be modified in various ways within the scope illustrated in the present disclosure, and embodiments obtained by appropriately combining the technical means disclosed in different embodiments are also included in the technical scope of the invention according to the present disclosure. In other words, note that a person skilled in the art can easily make various variations or modifications based on the present disclosure. Note that these variations or modifications are included within the scope of the present disclosure.

### REFERENCE SIGNS

1: Information processing system
10, 10A: Information processing device
20: Measuring device
30, 30X: Subject terminal
30A: Other-person terminal
31: Input reception unit
32: Presentation unit
101, 101X: Measurement result acquiring unit
102, 102X: History acquiring unit
104, 104X: Presentation information generation unit (Information generation unit)
105, 105X: Presentation instructing unit
110, 110A, 110X: Storage
111, 111X: Subject measurement result information
112, 112A, 112X: Subject history information
115, 115X: Presentation information data

## Claims

1. An information processing device, comprising:
a measurement result acquiring unit configured to acquire a first measurement result obtained by measuring a sample of a subject by using a measuring device;
a history acquiring unit configured to acquire a first history related to the subject; and
an information generation unit configured to generate presentation information to be presented to the subject by using the first measurement result, the first history, a second measurement result obtained by measuring a sample of another person different from the subject, and a second history related to the other person.

2. The information processing device according to claim 1, wherein
the information generation unit,
comprises a trained model having experienced a learning process based on a relationship between the second measurement result and the second history, and execution information related to a matter performed by the other person after acquisition of the second measurement result and the second history, and
generates the presentation information corresponding to the first measurement result and the first history by using the trained model.

3. An information processing device, comprising:
a measurement result acquiring unit configured to acquire a first measurement result obtained by measuring a sample of a subject by using a measuring device;
a history acquiring unit configured to acquire a first history related to the subject, the first history comprising a food purchase history related to food having been purchased by the subject; and
an information generation unit configured to generate presentation information to be presented to the subject by using the first measurement result and the first history.

4. The information processing device according to any one of claims 1 to 3,
the information processing device,
being capable of communicating with a subject terminal capable of inputting or measuring information to be comprised in the first history, and
comprising a presentation instructing unit configured to cause a presentation unit of the subject terminal to present the presentation information.

5. The information processing device according to any one of claims 1 to 4, wherein
the presentation information comprises an ingestion form of health food.

6. The information processing device according to any one of claims 1 to 5, wherein
the presentation information comprises at least any of information on food, information on exercises, and information on sleep.

7. The information processing device according to any one of claims 1 to 6, wherein
the information generation unit causes information indicating a time at which the subject preferably performs measurement using the measuring device to be in the presentation information.

8. The information processing device according to any one of claims 1 to 7, wherein
the information generation unit causes information indicating a method of measurement different from the measurement performed by the subject using the measuring device to be comprised in the presentation information.

9. The information processing device according to any one of claims 1 to 8, wherein
the presentation information also comprises information related to food that is specified based on the first history and is not related to the first measurement result.

10. The information processing device according to claim 1 or 2, wherein
the first history and the second history comprise a biological cycle comprising meal contents, meal times, exercise contents, exercise time, sleeping time, heart rate, body temperature, wake-up time, and bedtime of the subject or the other person.

11. The information processing device according to claim 1 or 2, wherein
the information generation unit causes information indicating a result of comparison between the measurement results of the subject and the other person to be comprised in the presentation information.

12. The information processing device according to claim 3, wherein
the information generation unit causes information recommending regular purchase of food presented to the subject to be comprised in the presentation information.

13. A subject terminal, comprising:
the information processing device according to any one of claims 1 to 12; and
a presentation unit configured to present the presentation information.

14. The subject terminal according to claim 13, further comprising:
a storage configured to store the first measurement result.

15. An information processing system, comprising:
the information processing device according to any one of claims 1 to 12; and
the measuring device, wherein
the measuring device and the information processing device are communicably connected through a wired or wireless dedicated line.

16. The information processing system according to claim 15, further comprising:
in the information processing device, a storage configured to store the first measurement result and a second measurement result obtained by measuring a sample of another person different from the subject.

17. A control method of an information processing system, the control method comprising:
acquiring a first measurement result obtained by measuring a sample of a subject by using a measuring device;
acquiring a first history related to the subject;
generating presentation information to be presented to the subject by using the first measurement result, the first history, a second measurement result obtained by measuring a sample of another person different from the subject, and a second history related to the other person; and
causing a presentation unit to present the presentation information.

18. A control method of an information processing system, the control method comprising:
acquiring a first measurement result obtained by measuring a sample of a subject by using a measuring device;
acquiring a first history related to the subject, the first history comprising a food purchase history related to food having been purchased by the subject;
generating presentation information to be presented to the subject by using the first measurement result and the first history; and
causing a presentation unit to present the presentation information.
